# EUROPÄISCHE PATENTANMELDUNG

(11) **EP 1 199 034 A2**
(43) Veröffentlichungstag der Anmeldung: **24.04.2002**
(21) Anmeldenummer: 01250369.4
(22) Anmeldetag: 19.10.2001
(51) Int. Cl.: A61B 8/12

(54) **Vorrichtung zur linearen und/oder rotatorischen Führung eines Gegenstandes wie einer Sonden- und Instrumentenführung**

(30) Priorität: 20.10.2000 DE 10051953
(71) Anmelder: jojumarie Intelligente Instrumente GmbH, 13125 Berlin (DE)
(72) Erfinder: Lüth, Tim, Prof., Dr., 13156 Berlin (DE)
(74) Vertreter: Hengelhaupt, Jürgen D., Dipl.-Ing.

(57) **Zusammenfassung**

Die Erfindung betrifft ein Verfahren und eine Vorrichtung zur linearen und/oder rotatorischen Führung eines Gegenstandes wie einer Ultraschallsonden- und Instrumentenführung zur intrakorporalen Anwendung.

Die Aufgabe der Erfindung, ein Verfahren und eine Vorrichtung zu entwickeln, mit welchen der Schutz des Patientengewebes vor Belastungen und Beschädigungen bei der manuellen oder automatischen Einführung von Ultraschallsonden und Instrumenten gewährleistet wird, die durch das Ausrichten und Bewegen der Sonde sowie bei Erschütterungen oder ungewollten Stößen gegen die Sonde oder das Instrument als auch deren Führungseinheiten entstehen, wird dadurch gelöst, dass der Gewebeschutzkörper (1) fest mit einem in seiner räumlichen Lage beliebig ausrichtbaren und reproduzierbaren und sicher zu fixierenden Geräteflansch (2) verbunden ist und dass in den Geräteflansch (2) Führungen für die Sonde (14) integriert sind und an dem Geräteflansch (2) Instrumente und/oder Instrumentenführungen leicht zu befestigen sind, deren intrakorporale Position durch die Ultraschallsonde (14) bestimmt werden kann (Instrumentenzielsystem 6).

## Beschreibung

Die Erfindung betrifft ein Verfahren und eine Vorrichtung zur linearen und/oder rotatorischen Führung eines Gegenstandes wie einer Sonden- sowie Instrumentenführung gemäß den Oberbegriffen der Ansprüche 1 und 2.

Die Vorrichtung ist insbesondere für Rektalsonden zur Prostatauntersuchung und -behandlung mittels Ultraschall geeignet.

Um in die Prostata schwachradioaktive Seeds einzubringen ist es erforderlich, unmittelbar vor der Implantierung die Lage und das Volumen der Prostata zu messen. Dies geschieht gegenwärtig mit Ultraschallsystemen, die Bilder mit einer Rektalsonde aufnehmen. Die Rektalsonde nimmt dabei Längs- oder Querschnitte des Gewebes oberhalb der Rektalsonde auf.

Um die Lage und das Volumen der Prostata relativ zu einem Referenzpunkt eines Behandlungsapparates außerhalb des Körpers zu bestimmen, wird eine Rektalsonde schrittweise über einen Linearvorschub in das Rektum geschoben. Nach jedem Schritt wird dabei ein Ultraschallbild des Gewebes aufgenommen. Ein Positionszähler gibt die räumliche Position der Sonde während der Bildakquisition wieder. Anschließend wird aus der Zuordnung der einzelnen Ultraschallbilder zu den Angaben des Positionszählers ein Schichtbildstapel analog zu einem Computertomographie-Bildstapel erzeugt. Durch eine manuelle Segmentierung der Ultraschallbilddaten und der manuellen Klassifizierung in einzelne Organe wird ein 3D-Modell der Prostata und anderer Organe erzeugt. Dabei wird für die Berechnung der Ausdehnung in den einzelnen Schichtbildern des Ultraschalls eine Umrechnung von Bildpunkten in ein Längenmaß herangezogen. Entweder wird dafür ein Ultraschallsystem mit bekanntem Umrechnungsfaktor verwendet oder im Ultraschallbild ist der Umrechnungsfaktor explizit enthalten.

Verschiedene manuelle, halb- und vollautomatische Vorschubsysteme (Stepper) für Ultraschallsonden im Rektalbereich sind bekannt.

Ein Vorschubsystem ist in der US 5,871,448 beschrieben. Es ist ein Stepper für das Halten und Bewegen einer Ultraschallsonde zur Bildaufnahme von inneren Organen, der eine Ultraschall-sonde aufnehmen und diese linear schrittweise oder frei verschieben kann.

Das bekannteste System für den manuellen schrittweisen Vorschub der Rektalsonde ist in der US 5,931,786 beschrieben. Es ist ein Apparat für das Führen medizinischer Instrumente, der aus einer Klammer zur Aufnahme von mindestens einem medizinischen Instrument, einer Einheit zur rotatorischen Bewegung dieser Klammer, einer Schiebevorrichtung zur Verschiebung der Gesamteinheit zu einem Bildaufnahmepunkt und einer Schnellkupplung zur Trennung von Klammer und Rotationseinheit besteht.

Bei beiden bekannten Systemen wird die Rektalsonde über eine Zahnstangenwinde mit einem Handrad in einem festen Vorschubraster verstellt. Die Kontrolle der Synchronisation von Bildaufnahme und Vorschub erfolgt manuell.

Es sind Verfahren und Systeme zum automatisierten Vermessen der Prostata bekannt.

Bei einem Verfahren zur Volumenmessung eines Tumors oder einer Drüse wird ein schnittbildgebender Sensor entlang einer Längsoder Rotationsachse bewegt (US 5,178,148). Die Bilder werden segmentiert und die Tumorgröße auf der Basis der Vorschubbewegung und der segmentierten Schnittbilder berechnet. Diese Methode ist in der gesamten Computertomographie bereits Stand der Technik.

Es sind mehrere Patente zu einem automatischen Stepper-Mechanismus bekannt (US 5,361,768, US 5,485,846, US 5,592,942, US 6,013,030).

Der Stepper besteht aus einem motorgetriebenen Linearantrieb zur schrittweisen Bewegung einer Ultraschallsonde, einer Vorrichtung zur Befestigung der Sonde, zwei Führungsschienen, zwei Spreizern zur Festlegung des Abstands der Führungsschienen. Der Vorschub erfolgt über eine Zahnstangenwinde, angetrieben von einem Motor über eine flexible Welle. Der Stepper hat darüber hinaus einen zweiten unabhängigen Linearantrieb zur Bewegung eines Hohlkatheters (US 5361768).

Aus der US 5,088,178 ist ein Ultraschall-Endoskop bekannt, welches mit einer dünnwandigen und ultraschalldurchlässigen Schutzhülle ausgestattet ist.

Aus der US 4,877,033 ist eine Untersuchungshülle für transvaginale Ultraschall-Untersuchungen bekannt.

Die bislang bekannten Behandlungseinheiten zur Vermessung der Prostata mit einer Rektalsonde und zur nadelgestützten SeedImplantierung werden im vollständig montierten Zustand an den Patienten herangeführt (US 5,931,786). Dabei muss berücksichtigt werden, dass sowohl automatisierte als auch manuelle Sonden-Vorschubmechanismen ein erhebliches Gewicht besitzen. Sie sind nicht geeignet, ohne gewichtskompensierende Positionierungsmechanismen eingesetzt zu werden. Über Mechanismen zur Grobpositionierung (Position und Orientierung) wird die Behandlungseinheit so ausgerichtet, dass die Ultraschallsonde in Richtung des Rektumausgangs zeigt. Einige Behandlungseinheiten erlauben die Fixierung der Behandlungseinheit an der Patientenauflage.

Bei der Ausrichtung der Rektalsondenführung linear in Richtung des Rektums ist zu berücksichtigen, dass der Verlauf des Rektums von außen nicht sichtbar und daher keine einfache lineare Ausrichtung möglich ist. In der Praxis ergibt sich hieraus eine wiederholt erforderliche Korrektur mit den teilweise vorhandenen Lage-Feinstellgliedern und einem Vor- und Zurückbewegen der Rektalsonde. Hierzu muss häufig die Grobpositionierung der gesamten Behandlungseinheit verändert werden, um so eine Ausrichtung der Rektalsondenführung zu erreichen, die Linearbewegungen im Rektum zulässt. Dies ist ein zeitaufwendiger Einstellungsprozess, der darüber hinaus bei unerfahrener Handhabung das Rektumgewebe erheblich belasten und verletzen kann.

Die Stabilisierung der Lage der Behandlungseinheit relativ zum Patienten ist bei den bekannten Systemen unzureichend. Das hohe Gewicht der Steppereinheiten kann durch die gegebene Fixierung mit langen Gelenkarm-Kinematiken nur unzureichend (relative Position und Orientierung zum Patientengewebe) stabilisiert werden. Dies ist besonders bei automatisierten vibrierenden Steppern zu beobachten.

Wird die Sonde mit Hilfe eines Vorschubs direkt oder indirekt (beispielsweise durch Wasserblasen, Gelkissen etc.) gegen das Gewebe gedrückt, wirken durch die direkte oder indirekte Interaktion Kräfte und Momente zwischen Sonde und Gewebe. Beim Vorschub einer Rektalsonde können bei einer ungünstigen Ausrichtung von Sonde und Rektum erhebliche Querkräfte auf das Rektum wirken. Dies ist umso kritischer, wenn das medizinische Instrument motorgetrieben und ohne direkte Kraftrückkopplung auf den behandelnden Arzt bewegt wird.

Liegen im Darminneren Polypen vor oder ist der Enddarm verengt, so können bei Nutzung der Rektalsonde die Polypen und das Rektumgewebe erheblich geschädigt oder überdeckt werden. Dieser Effekt wird durch ein wiederholtes Einführen der Sonde sowie eine Lagekorrektur verstärkt. Linearbewegungen der Rektalsonde erzeugen immer Gewebebelastungen, selbst wenn die Rektalsonde optimal ausgerichtet ist. Das Rektum muß erneut in Richtung der Sonde gedehnt, gestreckt und aphysisch bewegt werden.

Bei der Bewegung der Sonde kommt es immer zu einer Lageveränderung der Gewebestruktur. Dies führt während einer Behandlung mit eingestochenen Hohlnadeln zur Seedimplantierung zu einer kritischen Situation, da sich die Lage der Prostata relativ zum Ursprung der Bestrahlungsplanung verändert.

Aufgabe der Erfindung ist es, die bekannten Nachteile des Standes der Technik zu vermeiden und ein Verfahren und eine Vorrichtung zu entwickeln, mit denen der Schutz des Patientengewebes vor Belastungen und Beschädigungen bei der manuellen oder automatischen Einführung von Ultraschallsonden und Instrumenten gewährleistet wird, die durch das Ausrichten und Bewegen der Sonde sowie bei Erschütterungen oder ungewollten Stößen gegen die Sonde oder das Instrument als auch deren Führungseinheiten entstehen, sowie zu gewährleisten, dass die Bildaufnahmeebene eine Organs immer parallel und deckungsgleich zur Navigationsebene des Instrumentenzielsystems ausgerichtet ist.

Diese Aufgabe wird durch ein Verfahren nach den Merkmalen des Anspruchs 1 und eine Vorrichtung nach den Merkmalen des Anspruchs 2 gelöst.

Das Verfahren zur linearen und/oder rotatorischen Führung eines Bildaufnahmesystems wie eine Ultraschallsonde und/oder von Instrumenten zur intrakorporalen Anwendung wie zur transversalen Schnittbilderzeugung eines Organs, wobei das abzubildende Organ in seiner Lage stabil gehalten wird, ist dadurch gekennzeichnet, dass zwischen dem Bildaufnahmesystem und einem Zielsystem für das Organ eine feste Verbindung hergestellt wird, die sicherstellt, dass die Navigationsebene des Zielsystems parallel und deckungsgleich zu den Bildebenen verläuft.

Die Vorrichtung zur linearen und/oder rotatorischen Führung eines Gegenstandes wie einer Ultraschallsonden- und Instrumentenführung zur intrakorporalen Anwendung, mit einem den Ultraschall leitenden, formstabilen, dünnwandigen, hohlen Gewebeschutzkörper ist dadurch gekennzeichnet, dass der Gewebeschutzkörper fest mit einem in seiner räumlichen Lage beliebig ausrichtbaren und reproduzierbaren und sicher zu fixierenden Geräteflansch verbunden ist und dass in den Geräteflansch Führungen für die Ultraschallsonde integriert sind und an dem Geräteflansch Instrumente und/oder Instrumentenführungen leicht zu befestigen sind, deren intrakorporale Position durch die Ultraschallsonde bestimmt werden kann (Instrumentenzielsystem).

Die Vorrichtung ist insbesondere für Rektalsonden zur Prostatauntersuchung und -behandlung mittels Ultraschall vorteilhaft anwendbar.
Das Rektum des Patienten wird nur ein einziges Mal während der gesamten Behandlung (Sondenausrichtung, Prostatavermessung, ultraschallgestützte Nadelführung, kontinuierliche Bestrahlungsplanung) gedehnt und verbleibt bis zum Ende der Behandlung in diesem Zustand. Dadurch wird die Belastung des Rektums und des umliegenden Gewebes minimiert.

Die Ultraschallsonde kann zusammen mit dem sehr leichten Gewebeschutzkörper mit einer Hand gegriffen, in das Rektum eingeführt und manuell optimal ausgerichtet werden.

Nur eine Hand ist zur Fixierung des Gewebeschutzkörpers relativ zum Patienten an der Patientenauflage erforderlich. Die Positionierung erfolgt unter kontinuierlicher Kontrolle mittels Ultraschallaufnahmen. Der behandelnde Arzt hat die Sicherheit, dass die gewünschte Ausrichtung in kurzer Zeit gefunden und durch Fixierung gesichert wird.
Alle weiteren und gegebenenfalls schweren Geräte werden erst nach sicherer Lagefixierung des Gewebeschutzkörpers und des Geräteflansches montiert und können keine weiteren Gewebebelastungen mehr hervorrufen.

Die Geräte können wiederholbar mit maximaler Genauigkeit relativ zum Referenzpunkt des Geräteflansches bzw. den im Ultraschall sichtbaren Markierungen am Gewebeschutzkörper angebracht werden.

Durch die im Ultraschall sichtbaren Markierungen auf dem Gewebeschutzkörper kann der Arzt direkt im Ultraschallbild erkennen, wie tief vom Lagereferenzkörper entfernt und in welcher Richtung sich Gewebestrukturen, wie beispielsweise die Prostata, befinden. Dies unterstützt diagnostische und therapeutische Entscheidungen.

Durch die im Ultraschall sichtbaren Markierungen erhält man für die Instrumentenführung sowohl einen absoluten Tiefen- und Orientierungsmaßstab als auch einen geweberelativen Abstandsund Winkelmaßstab.

Die in bekannten Abständen angebrachten Markierungen erlauben eine Winkel- und Abstandskalibrierung der Messsonde bzw. des Ultraschallbildes und können als zusätzliches Meßsystem bei bild-, computer- und robotergestützten Eingriffen genutzt werden.

Apparaturen zur Führung der Sonde oder von Instrumenten können schnell an- und abgebaut werden, ohne dass die Lagereferenzierung verloren geht. Die Behandlung kann nach einer Unterbrechung fortgesetzt werden.

Der Geräteflansch mit offener Achsenführung erlaubt die Anbringung unterschiedlichster manuell oder automatisch angetriebener Sonden- und Instrumentenführungen.

Durch die Aufnahme der Instrumentenführung direkt am Gewebe-schutzkörper und die im Ultraschall sichtbaren Markierungen gelten alle vorherigen Vorteile, die sich aus Lagereferenzkörper und Markierungen ergeben auch für das Einbringen von Instrumenten, die im Ultraschallkegel sichtbar sind, in das Gewebe außerhalb des Gewebeschutzkörpers.

In einer bevorzugten Ausführungsform der Vorrichtung bildet der Gewebeschutzkörper die Außenwand der Ultraschallsonde, wobei ein bildgebender Teil der Ultraschallsonde in dem Gewebeschutzkörper axial verfahrbar und rotierbar ist und wobei eine feste Kopplung zwischen der Außenwand und dem Geräteflansch sowie dem Instrumentenzielsystem besteht.

Weitere vorteilhafte Ausführungsformen sind in den Unteransprüchen beschrieben.

Nachfolgend wird in einem Ausführungsbeispiel eines Gewebeschutzkörpers für die Einführung einer Rektalsonde zur Prostatauntersuchung die Erfindung näher erläutert. In der zugehörigen Zeichnung zeigen:
- Fig. 1 :: die schematische Darstellung eines Gewebeschutzkörper mit Geräteflansch und Fixierung,
- Fig. 2 :: die Anordnung von Gewebeschutzkörper mit Ultraschallsonde im Körper,
- Fig. 3 :: den Gewebeschutzkörper gemäß Fig. 1 mit Druckmembranen,
- Fig. 4 :: die schematische Darstellung einer Patientenauflage zur Fixierung des Gewebeschutzkörpers und des Geräteflansches,
- Fig. 5 :: eine alternative Ausführungsform des Gewebeschutzkörpers und
- Fig. 6 :: der Gewebeschutzkörper nach Fig. 1 mit Gleitschale.
- Fig. 7 :: die schematische Darstellung einer Ausführungsform, in welcher die Außenwand der Ultraschallsonde durch den Gewebeschutzkörper gebildet ist.

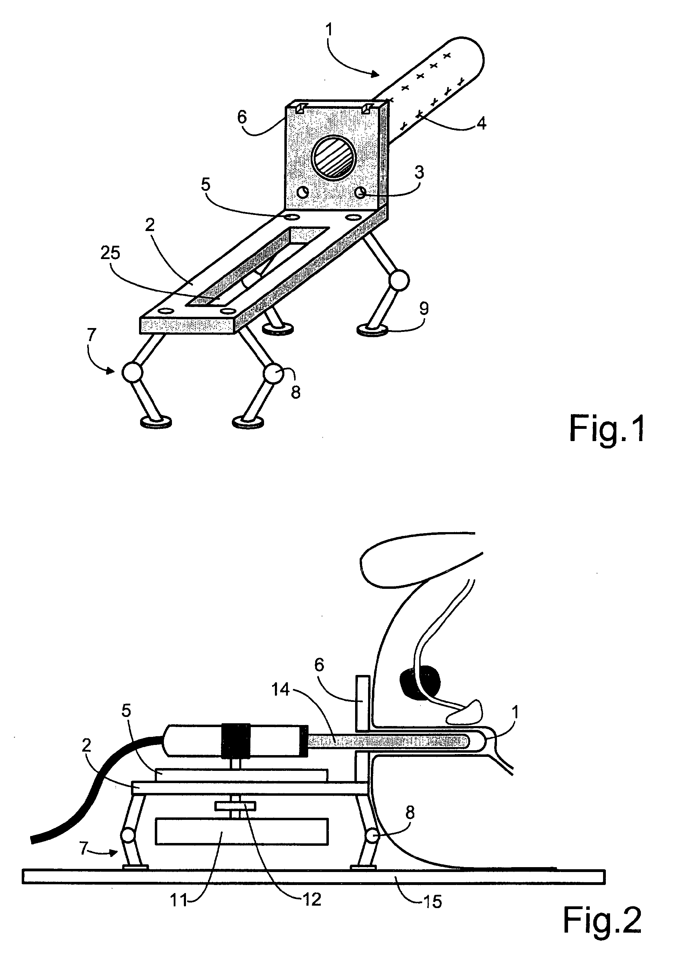

Fig. 1 zeigt einen Gewebeschutzkörper 1 und einen mit ihm fest verbundenen Geräteflansch 2. Der Geräteflansch 2 wird durch die Fixierungen 7, 8 in seiner Lage (Position und Orientierung) ausgerichtet und fixiert. In den Gewebeschutzkörper 1 ist eine durch Lagereferenzkörper 3 definierte Geometrie eingebracht, die ein Referenzkoordinatensystem aufspannt.

Die Geometrie und Lage aller Instrumenten- und Sondenführungen sowie Maßstabsmarkierungen 4 am Gewebeschutzkörper 1 sind relativ zu dem Lagereferenzkörper 3 bekannt. Die Markierungen 4 bilden einen im Ultraschall sichtbaren Längen- und Winkelmaßstab.

Der Geräteflansch 2 ist eine leichte, steife Struktur und besitzt Öffnungen 25 (Kupplungsdurchführungen) in den kartesischen Orientierungen, um Antriebsachsen oder Kupplungen durch diese zu führen. Dies ermöglicht das Anbringen einer leichten Sondenführung 5 oberhalb des Geräteflansches 2 in der Längsachse des Gewebeschutzkörpers 1 und des Antriebs dieser Sondenführung 5 von unterhalb des Geräteflansches 2. Der Geräteflansch 2 verfügt über kalibrierte Vorrichtungen zur Aufnahme von Sonden- sowie Instrumentenführungen 5. Diese sind ein Instrumentenführungsflansch 6 zur Aufnahme von kalibrierten Instrumentenführungen (z.B. Nadelführungsgitter) und ein Sondenführungsflansch zur Aufnahme von kalibrierten Sondenführungen.

Jede Flanschfixierung 7 hat sechs Freiheitsgrade. Die Freiheitsgrade der Flanschfixierung 7 können mit mindestens einem Fixierungshebel 8 oder einem Fixierungsrad an ihren Fußpunkten 9 fixiert werden. Dies kann auch für alle Flanschfixierungen 7 über einen zentralen Fixierungshebel (hydraulisch oder mit einem Baudenzug) erfolgen. Die Flanschfixierungen 7 sind so ausgelegt, dass ein Halten des Geräteflansches 2 und aller an diesem befestigten Elemente mit hoher Lagestabilität möglich ist.

Der Gewebeschutzkörper 1 ist ein dünnwandiger ultraschall-leitender Hohlkörper mit mindestens einem Lumen zur Aufnahme einer Rektalsonde. Er ist in Einschubrichtung vorn geschlossen. Alle eventuell vorhandenen Kanten müssen ausreichend große Radien besitzen, um Verletzungen auszuschließen. Auf dem Gewebeschutzkörper 1 sind in Längsrichtung und radial im Ultraschall und im Röntgenbild sichtbare Markierungen 4 mit bekannter Lage (Position und Orientierung) relativ zum Lagereferenzkörper 3 implementiert.

Fig. 2 zeigt die Anordnung von Gewebeschutzkörper 1 und Geräteflansch 2 mit montierter Instrumentenführung 6, Sondenführung 5, Antriebsachse 10, Kupplung 12 und Antrieb 11 sowie der Ultraschallsonde 14 bei einer Fixierung über die Flanschfixierung 7 an einer Patientenauflage 15 bzw. den Montageflächen 21. Die Fixierung des Antriebs 11 ist nicht eingezeichnet.

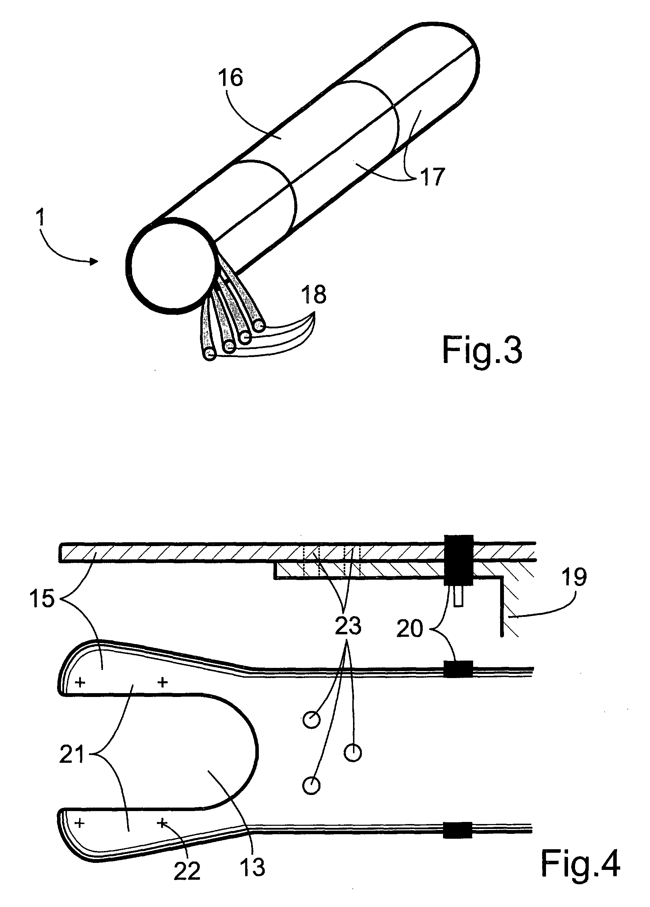

Fig. 3 zeigt eine aufpumpbare Membran 16 (Luftballon, Kondom), die auf dem Gewebeschutzkörper 1 angebracht ist. Die Membran 16 ist in unterschiedliche Segmente 17 aufgeteilt, die einzeln über jeweils eine Druckzufuhr 18 aufgepumpt werden. Es kann auch eine Membran 16 verwendet werden, die ein vorbestimmtes Profil in Abhängigkeit von dem jeweils gültigen Druck auf diese besitzt. Dann kann das Profil über eine Druckzufuhr nach Bedarf eingestellt werden.

Fig. 4 zeigt die Patientenauflage 15 in einer schematischen Seitenansicht und Draufsicht, die auf eine Behandlungsliege 19 aufgelegt und über einen Fixiermechanismus 20 mit dieser verbunden ist. Die relative Position von Patientenauflage 15 und Behandlungsliege 19 ist durch überführbare Geometrien von Lagereferenzkörpern 23 in der Patientenauflage 15 und der Behandlungsliege 19 bekannt.

Die Patientenauflage 15 besitzt sehr steife ausgestellte Montageflächen 21, die über die Grundplatte der Behandlungsliege 19 herausragen und für die stabile Aufnahme von Arretierungen und Abstützungen konzipiert sind. In die Patientenauflage 15 sind Lagereferenzkörper 23 eingearbeitet, die ein eindeutiges Referenzkoordinatensystem der Patientenauflage 15 bilden. Die genaue Geometrie des Lagereferenzkörpers 23 ist bekannt. Ein Lagereferenzkörper 23 ist in bildgebenden Verfahren (Ultraschall und Röntgen bzw. Computertomographie) erkennbar.

Die in bildgebenden Verfahren lokalisierbaren und messbaren Lagereferenzkörper 23 sind dazu geeignet, Patientenfixierungen und Geräteflansche mit hoher Lagegenauigkeit an der Patientenauflage 15 zu befestigen. Die Patientenauflage 15 ist aus einem Material hergestellt, dass keine Artefakte in bildgebenden Systemen hervorruft.

Der Patient liegt entweder direkt auf der Patientenauflage 15 oder diese ist stabil an der Behandlungsliege 19 befestigt und verlängert diese. An den Montageflächen 21 sind die Fußpunkte 9 der Flanschfixierung 7 angebracht (Fig. 1). Die Patientenauflage 15 ist aus einem hochfesten und hochsteifen Material hergestellt, um eine maximale Positions- und Lagestabilität zu erzielen. Sie ist ausreichend groß, um den Patienten direkt auf ihr zu lagern. Die Patientenauflage 15 kann beheizt sein. Die Aussparung 13 zwischen den Montageflächen 21 ist möglichst groß zu wählen, so dass der Arbeitsraum des Arztes nicht eingeschränkt wird. Die Montageflächen 21 ragen über die ursprüngliche Breite der Behandlungsliege 19 hinaus. Sie können Vorrichtungen oder Fußpunkte zur Lagerung der Patientenbeine tragen.

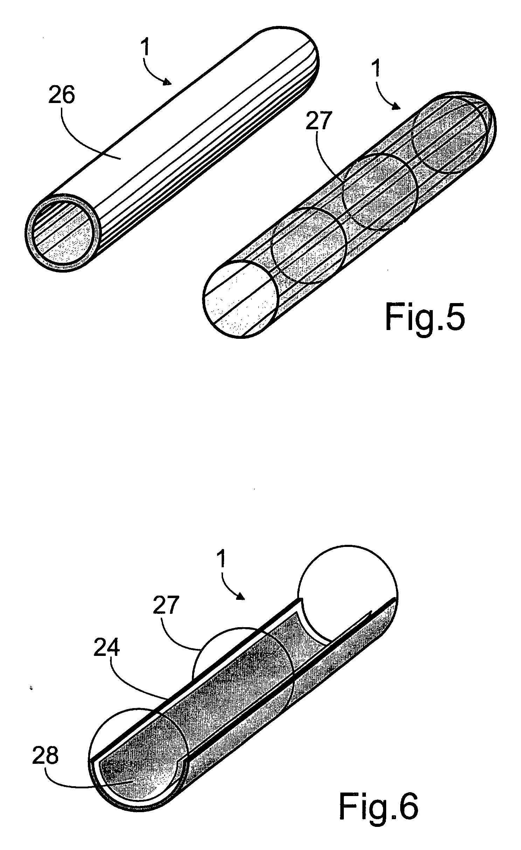

Fig. 5 zeigt Ausführungsformen des Gewebeschutzkörpers 1 als Vollkörper 26 und eine alternative Form, bestehend aus einer gitterartigen Struktur 27, welche mit einer flexiblen Folie bespannt ist. Alle Materialien leiten den Ultraschall.

In Fig. 6 ist in den Gewebeschutzkörper 1 eine Gleitschale 24 zur radialen Lagerung und Führung der Sonde 14 eingebettet. Diese verhindert einen zu starken Verlust an Gleitmittel im Spalt zwischen Sonde 14 und Gewebeschutzkörper 1. Innerhalb der Gleitschale 24 ist ein Schlitten 28 vorgesehen.

Zunächst wird die Patientenauflage 15 in kalibrierter Lage auf die Behandlungsliege 19 aufgelegt und mit dem Fixierungs-mechanismus 20 befestigt. Dann wird der Patient auf der Patientenauflage 15 geeignet gelagert und fixiert. Anschließend wird der Gewebeschutzkörper 1 mit einem ultra-schallleitenden Gleitmittel eingerieben und mit der Hand in das Rektum eingeführt. Dabei kann sich in dem Gewebe-schutzkörper 1 bereits die Ultraschallsonde 14 befinden. Ist der Gewebeschutzkörper 1 soweit eingeführt, dass die Prostata im Ultraschall optimal sichtbar ist, wird der Gewebeschutz-körper 1 zusammen mit dem Geräteflansch 2 in der gewünschten Lage mit den Fixierungselementen 20 an der Patientenauflage 15 lagestabil befestigt. Dabei können die Ultraschallsonde, der Geräteflansch 2 und der Gewebeschutzkörper 1 mit einer Hand gehalten werden. Mit der anderen Hand oder über einen Fußschalter wird deren Lage (Position und Orientierung) fixiert. Das Rektum des Patienten sowie die inneren Organe, wie z.B. die Prostata, sind somit im Referenzkoordinatensystem des Geräteflanschs 2 beschrieben.
Mit der Druckmembran 16 kann nun das Gewebe oberhalb des Gewebeschutzkörpers 1 so angehoben werden, dass das gewünschte Gewebe im Ultraschallkegel ausreichend sichtbar wird.
Nun werden die Sonden- und Instrumentenführungen und die Antriebselemente angebracht. Durch die Öffnungen des Geräteflansches 2 werden die Antriebselemente geführt.
Die Ultraschallsonde 14 ist an der Sondenführung befestigt. Die Ultraschallsonde 14 kann jetzt manuell oder motorisch in dem Gewebeschutzkörper 1 bewegt werden, ohne daß es zu weiteren Belastungen oder Verletzungen des Gewebes als auch Gewebeverschiebungen durch die Bewegung der Sonde 14 kommt. Auch kann die Ultraschallsonde 14 entfernt und erneut mit ultraschallleitendem Gleitmittel versehen werden, ohne dass die Referenzposition verloren geht. Weitere Diagnose- und Therapiegeräte können ebenfalls durch den Gewebeschutzkörper 1 bzw. das Referenzkoordinatensystem referenziert und wiederholbar positioniert werden.

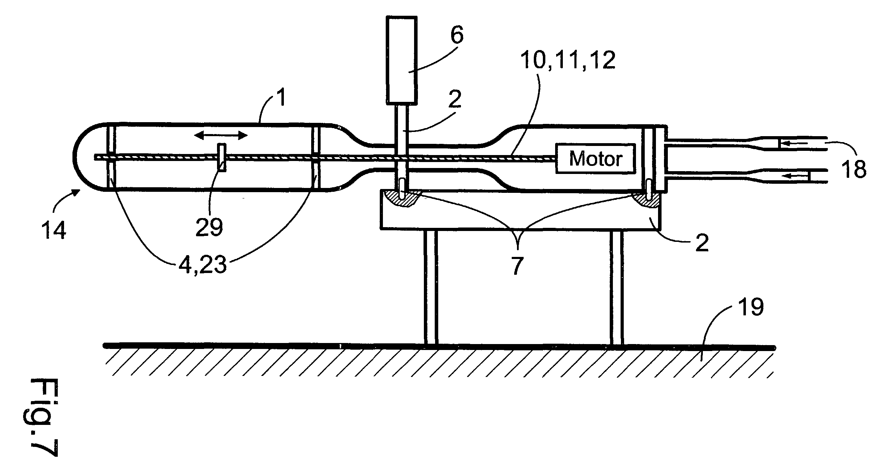

In der Fig. 7 ist eine Ausführungsform der Vorrichtung dargestellt, bei der die Außenwand der Ultraschallsonde 14 durch den Gewebeschutzkörper 1 gebildet ist.

Die Ultraschallsonde 14 weist einen bildgebenden Teil 29 auf, welcher in der Ultraschallsonde 14 axial verfahrbar und rotierbar ist. Die Ultraschallsonde 14 ist dabei über ihre Außenwand 1 fest mit dem Geräteflansch 2 und dem Instrumentenzielsystem 6 verbunden.

Durch die Herstellung einer festen Verbindung zwischen dem Bildaufnahmesystem, realisiert durch die Ultraschallsonde 14 mit dem bildgebenden Teil 29, und dem Zielsystem, realisiert durch den Instrumentenführungsflansch 6 mit den Lagereferenzkörpern 3 für das Organ, ist sichergestellt, dass die Navigationsebene des Zielsystems 6 parallel und deckungsgleich zu den nicht dargestellten Bildebenen des Organs verläuft.

Das Instrumentenzielsystem 6 muss in allen Ausführungsformen so positioniert sein, dass die jeweilige Bildaufnahmeebene des Organs parallel und deckungsgleich zur Navigationsebene des Zielsystems 6 ausgerichtet ist.

### Bezugszeichenliste

- 1: Gewebeschutzkörper
- 2: Geräteflansch
- 3: Lagereferenzkörper (Geometrie)
- 4: Maßstabmarkierungen
- 5: Sondenführungsaufnahme
- 6: Instrumentenführungsflansch (Instrumentenzielsystem)
- 7: Flanschfixierung
- 8: Fixierungshebel
- 9: Fußpunkt
- 10: Antriebsachse
- 11: Antrieb
- 12: Kupplung
- 13: Aussparung
- 14: Ultraschall-Sonde
- 15: Patientenauflage
- 16: Membran
- 17: Segment
- 18: Druckzufuhr
- 19: Behandlungsliege
- 20: Auflagen-Fixiermechanismus
- 21: Montagefläche
- 22: Aufnahme
- 23: Auflagen-Lagereferenzkörper
- 24: Gleitschale
- 25: Öffnung
- 26: Vollkörper
- 27: Gitterartige Struktur
- 28: Schlitten
- 29: Bildgebender Teil von 14

## Patentansprüche

1. Verfahren zur linearen und/oder rotatorischen Führung eines Bildaufnahmesystems wie eine Ultraschallsonde und/oder von Instrumenten zur intrakorporalen Anwendung wie zur transversalen Schnittbilderzeugung eines Organs, wobei das abzubildende Organ in seiner Lage stabil gehalten wird, **dadurch gekennzeichnet, dass** zwischen dem Bildaufnahmesystem (14) und einem Zielsystem (6) für das Organ eine feste Verbindung hergestellt wird, die sicherstellt, dass die Navigationsebene des Zielsystems (6) parallel und deckungsgleich zu den Bildebenen verläuft.

2. Vorrichtung zur linearen und/oder rotatorischen Führung einer Ultraschallsonde und/oder eines Instrumentes zur intrakorporalen Anwendung, mit einem den Ultraschall leitenden, formstabilen, dünnwandigen, hohlen Schutzkörper, **dadurch gekennzeichnet, dass** der Gewebeschutzkörper (1) fest mit einem in seiner räumlichen Lage beliebig ausrichtbaren und reproduzierbaren und sicher zu fixierenden Geräteflansch (2) verbunden ist und dass in den Geräteflansch (2) Führungen für die Sonde (14) integriert sind und an dem Geräteflansch (2) Instrumente und/oder Instrumentenführungen leicht zu befestigen sind, deren intrakorporale Position durch die Ultraschallsonde (14) bestimmt werden kann (Instrumentenzielsystem (6)).

3. Vorrichtung nach Anspruch 2, **dadurch gekennzeichnet, dass** der Gewebeschutzkörper (1) aus einer bespannten Gitterstruktur oder einem eckigen Körper mit abgerundeten Kanten jeweils mit geringer Wanddicke gebildet ist.

4. Vorrichtung nach Anspruch 2, **dadurch gekennzeichnet, dass** auf dem Gewebeschutzkörper (1) im Ultraschallbild sichtbare Markierungen (4) radial und linear in bekannten Abständen angebracht sind.

5. Vorrichtung nach den Ansprüchen 2 bis 4, **dadurch gekennzeichnet, dass** auf oder um den Gewebeschutzkörper (1) eine Membran (16) mit mindestens einer Öffnung vorgesehen ist, über die ein den Ultraschall leitendes Medium eingebracht und Gewebe oberhalb des Gewebeschutzkörpers (1) bewegt werden kann.

6. Vorrichtung nach den Ansprüchen 2 bis 5 , **dadurch gekennzeichnet, dass** die Ultraschallsonde (14) und deren manuelle oder motorische Antriebe nachträglich montierbar sind.

7. Vorrichtung nach den Ansprüchen 2 bis 6, **dadurch gekennzeichnet, dass** die im Ultraschall sichtbaren Markierungen (4) in bekannten Abständen zum Referenzkörper (3) aufgebracht, jedoch nicht in allen Quer- und Längsschnitten des Ultraschalls sichtbar sind, sondern nur in zwei aufeinanderfolgenden Abständen zu erkennen sind, dann jedoch ihre Position wechseln.

8. Vorrichtung nach den Ansprüchen 2 bis 7, **dadurch gekennzeichnet, dass** die Markierungen (4) auch in Röntgenbildern sichtbar sind.

9. Vorrichtung nach den Ansprüchen 2 bis 8, **dadurch gekennzeichnet, dass** eine Gelpumpe zur Zuführung von Gel in den Spalt zwischen dem Gewebeschutzkörper (1) und der Sonde (14) vorgesehen ist.

10. Vorrichtung nach den Ansprüchen 2 bis 9, **dadurch gekennzeichnet, dass** die Außenwand der Ultraschallsonde (14) durch den Gewebeschutzkörper (1) gebildet ist, wobei ein bildgebender Teil der Ultraschallsonde (14) in dem Gewebeschutzkörper (1) axial verfahrbar und rotierbar ist und wobei eine feste Kopplung zwischen der Außenwand der Ultraschallsonde (14) und dem Geräteflansch (2) sowie dem Instrumentenzielsystem (6) besteht.
